Europäisches Patentamt

European Patent Office

Office européen des brevets

(19)

(11) Publication number: **0 370 962**
**A2**

## (12) EUROPEAN PATENT APPLICATION

(21) Application number: 89830369.8

(51) Int. Cl.⁵: **A61F 13/12**

(22) Date of filing: **25.08.89**

The title of the invention has been amended (Guidelines for Examination in the EPO, A-III, 7.3).

(30) Priority: **29.09.88 IT 4840188**
**25.05.89 IT 4799189**

(43) Date of publication of application:
**30.05.90 Bulletin 90/22**

(84) Designated Contracting States:
**AT BE CH DE ES FR GB GR LI LU NL SE**

(71) Applicant: **Millauro, Carlo**
**Via A. Richelmy, 38**
**I-00165 Roma(IT)**

(72) Inventor: **Millauro, Carlo**
**Via A. Richelmy, 38**
**I-00165 Roma(IT)**

(74) Representative: **Massari, Marcello**
**Studio M. Massari S.r.l. 23, Via Fontanella**
**Borghese**
**I-00186 Roma(IT)**

(54) **Hygienic protection for tracheotomized patients.**

(57) A hygienic bib comprising a double absorbing pad and a removable and replaceable outer cloth intended to be used by tracheotomized patients, having a tracheal cannula, pivotally supported by a plate, inserted in an air passage made at the neck base, comprising a flat rectangular support, having a hole for accomodating the cannula and provided with strips for tying the bib around the patient's neck, which strips are provided with a reusable contact quick joining means, the support comprising a first face of a waterproof material and a second face of a permeable, loose woven, not woven-fabric, joined to the first face along the edges thereof, a layer of a spongy or absorbing material being interposed therebetween; an outer permeable cloth having the same size and being preferably made of the same fabric as the second face of the support and two laces for joining the support to the plate of the cannula. The bib is completed by a multilayer pad, preferably of the same permeable fabric as the outer cloth, having the shape of an elongated stripe and presenting a hole for accomodating the cannula, intended to be placed between the patient's skin and the waterproof face of the rectangular support.

*FIG.3*

## A HYGIENIC BIB COMPRISING A DOUBLE ABSORBING PAD AND A REMOVABLE AND REPLACEABLE OUTER CLOTH INTENDED TO BE USED FOR TRACHEOTOMIZED PATIENTS

A hygienic bib comprising a double absorbing pad and a removable and replaceable outer cloth intended to be used by tracheotomized patients, having a tracheal cannula, pivotally supported by a plate, inserted in an air passage made at the neck base, comprising a flat rectangular support, having a hole for accomodating the cannula and provided with strips for tying the bib around the patient's neck, which strips are provided with a reusable contact quick-joining means, the support comprising a first face of a waterproof material and a second face of a permeable, loose woven, fabric joined to the first face along the edges thereof, a layer of a spongy or absorbing material being interposed therebetween; an outer permeable cloth having the same size and being preferably made of the same fabric as the second face of the support and two laces for joining the support to the plate of the cannula. The bib is completed by a multilayer pad preferably of the same permeable fabric as the outer cloth, having the shape of an elongated stripe and comprising a hole for accomodating the cannula, intended to be placed between the patient's skin and the waterproof face of the rectangular support.

As it is known, patients subjected to tracheotomy breathe through a passage or channel having the mouth or inlet hole at the base of the patient's neck.

A short length of a cannula of a biocompatible material protrudes from this hole, the cannula being fitted within the channel to maintain it pervious.

The position of the hole is such that it is necessary to use an element which, yet covering the hole, allows the air to pass therethrough. Furthermore its nature is such that, while this element must be able to absorb the loss of serum and other substances coming out of the hole, the visible portion thereof must always maintain a clean and tidy aspect, in spite of the losses and its absorbing function.

Accordingly, this invention is intended to provide such an element in form of a bib that can be easily tied around the patient's neck and substantially comprises, as mentioned above, three elements which together brilliantly overcome the above-mentioned drawbacks.

To this aim the first absorbing pad and the outer cloth are two separate members in respect of the bib.

As mentioned above, the position and nature of the inner pad and the rectangular support are such that they are provided with a hole having a diameter slightly larger than the diameter of the cannula, whereas the outer cloth, that has mainly an aesthetic fuction and absorbing nature, is unprovided with such a hole.

The outer cloth is joined to the outer face, that is the second permeable face of the support through a contact joining means, other contact joining means being placed also on the two strips for tying the bib around the patient's neck.

This means may comprise snap fasteners or, as an alternative, small disks or pieces of a sheet of a bioadhesive material.

Furthermore the bib has two quick-joined laces to tie up the bib to the cannula for preventing the cannula from being thrown out of the passage, for instance owing to a fit of coughing.

The bib of the invention will be now described in detail with reference to the annexed drawings, wherein:

fig. 1 is a view of the outer portion of the bib, the cannula having been omitted, wherein the outer protecting cloth partially is raised and the corner of the outer face partially cut away to show the inner absorbing pad;

fig. 2 is a similar view of the rear portion of the bib, wherein the outer pad is partially cut away to show the laces for tying up the bib to the cannula plate;

fig. 3 is a vertical section, showing fragmentary and in a larger scala the main portion of the bib and separate outer pad;

fig. 4 is a perspective view showing, fragmentary and in a larger scale, the cannula plate and tying laces;

fig. 5 is a fragmentary section, in a larger scale, taken along the cannula axis;

fig. 6 is a fragmentary, top view of the sectioned plate and tying laces;

fig. 7 show a bib having alternative means for joining the two strips behind the patent neck.

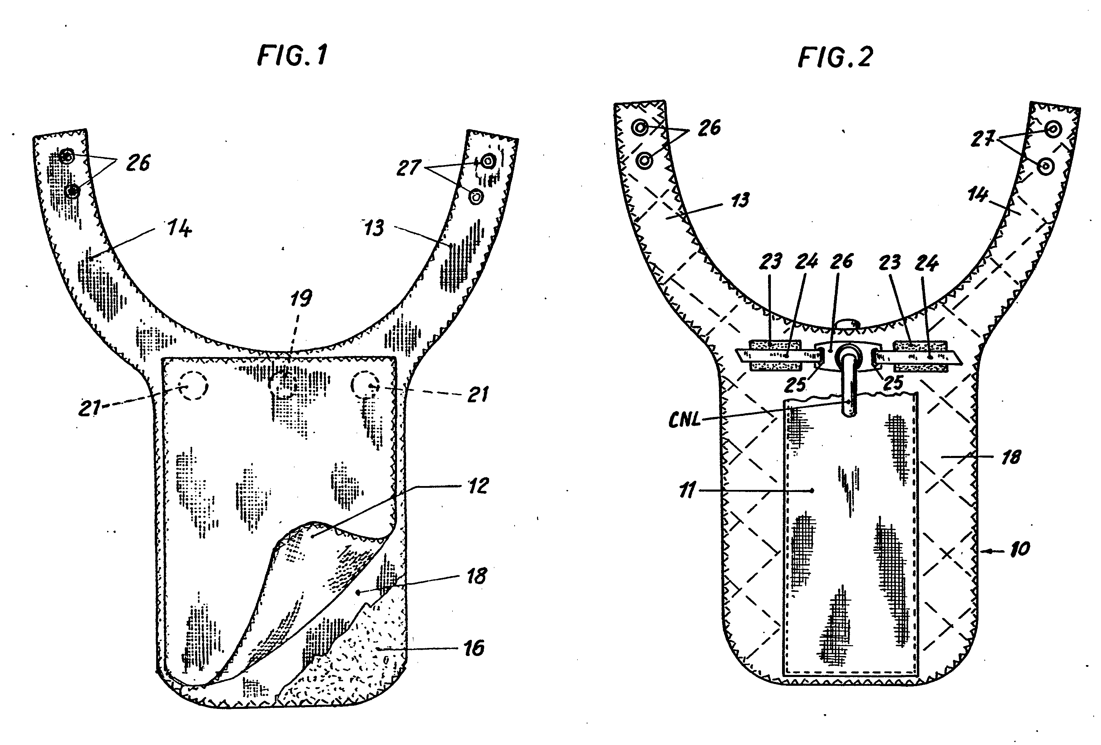

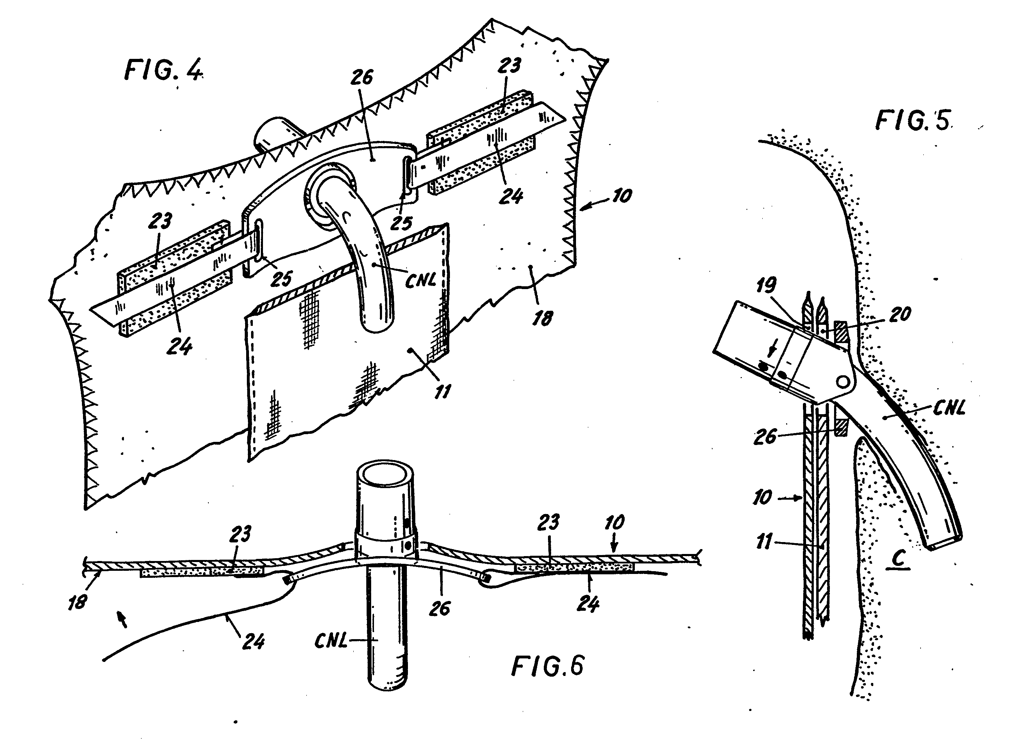

Referring firstly to figs. 1, 2 and 3, reference 10 indicates the rectangular support having rounded corners, reference 11 indicates the separate absorbing pad and reference 12 indicates the outer cloth while references 13 and 14 indicate the tying strips integrally formed with support 10 for tying the bib to the patient's neck.

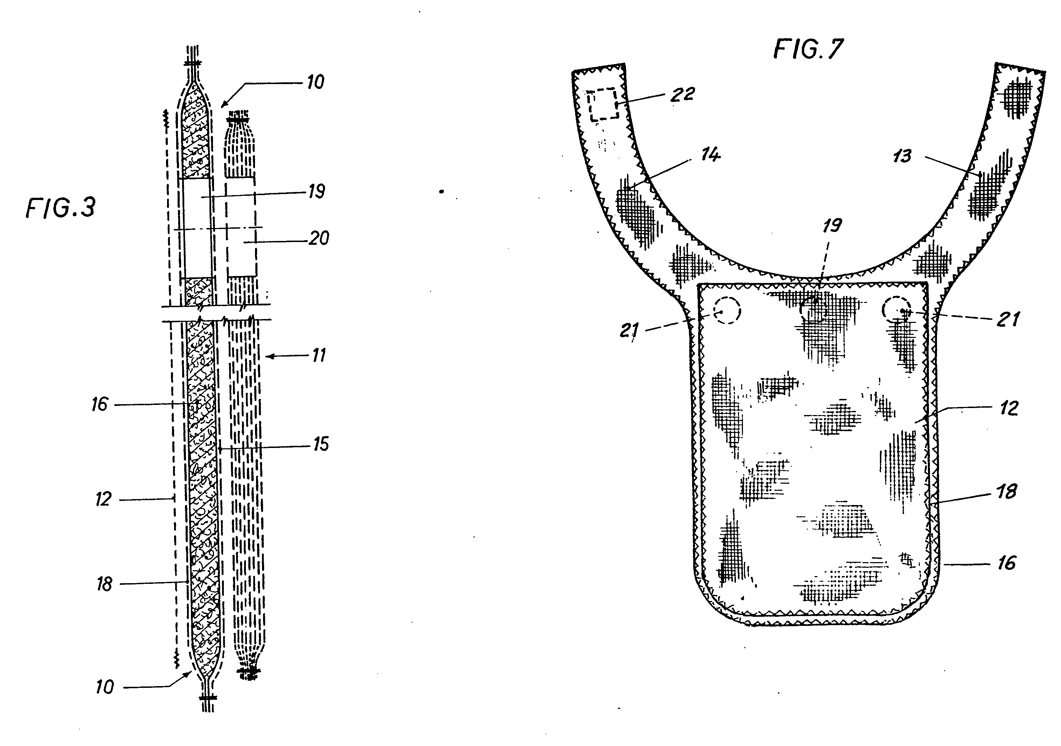

Referring now to the section of fig. 2, support 20 comprises a first face, i.e. the inner face of the bib when tied around the patent's neck, made of a waterproof material and referred to by numeral reference 15; a padding portion made of a spongy, adsorbing material comprising the inner adsorbing pad, referred to by numeral reference 16 and a second face, i.e. the outer face made of a permeable material as a loose woven fabric, referred to by numeral reference 18.

As clearly shown, both support 10 and pad 11 have a central hole formed in the upper portion thereof, the two holes, which are referred to by numeral references 19 and 20, respectively correspond to each other and have the same diameter.

As mentioned above, these two holes will be fitted over the outer end of cannula CNL protruding from the mouth of the channel made at the base of the patient's neck C to allow tracheotomized patients to breathe (fig. 4).

Outer cloth 12, intended to cover the outer end of cannula CNL, has no hole and presents at each corner a small disk of a material joining by contact, as a disk made of a biadhesive sheet material, as indicated by numeral reference 21.

Disks 21 allow outer cloth 12 to be easily sticked to outer face 18 of support 10 and separated therefrom, to be substituted when necessary.

Two rectangular pieces of the "velvet" portion of the material known under trademark VELCRO are sewn or otherwise suitably attached to the rear face 18 of support 10, these pieces comprising, as well known, a very large number of small loops.

These rectangular pieces are indicated by numeral references 23 and to the inner portion of each of the pieces 23, the end of a folded lace 24 is joined, made of the matching portion of the VELCRO material; accordingly these laces comprise a strip of the VELCRO portion bearing the hooks.

Strips 24 are as wide as to pass into slots 25 formed at the ends of plate 26 on which cannula CNL is pivoted.

Strips 24 fitted into slots 23, folded and attached to pieces 23, as shown in figures 2, 3 and 5, are intended to tie up cannula CNL to the bib which, in turn, is tied around the neck C of the tracheotomized patient through strips 13 and 14. Strips 12 and 14 are joined to each other by means of the two pairs of snap fasteners 26 and 27.

As an alternative, strips 13 and 14 may be joined together by means of a small square piece 22 of a biadhesive sheet material as shown in the fragmentary view of fig. 7.

As it will be evident, besides an indoubitable usefulness in its function as element of tidiness in tracheotomized patients making use of a cannula, the bib of the invention has also the advantage of allowing cannula CNL to be kept in place against any expulsive force, as for instance a fit of coughing.

As regards the used materials, waterproof face 15 can be made of a hypoallergenic synthetic material such as polyethylene, padding 16 can be made of cellucotton or pure cellulose and outer face 18 will preferably be of gauze.

Outer cloth 12 and the outer faces and inner layers of rear pad 11 will also preferably made of gauze.

Faces 15 and 18 of support 10 will be preferably joined together by sewing.

## Claims

1. A hygienic bib comprising a double absorbing pad and a removable and replaceable outer cloth intended to be used by tracheotomized patients, having a tracheal cannula, pivotally supported by a plate, inserted in an air passage made at the neck base, comprising a flat rectangular support, having a hole for accomodating the cannula and provided with strips for tying the bib around the patient's neck, which strips are provided with a reusable contact quick joining means, the support comprising a first face of a waterproof material and a second face of a loose-woven, permeable fabric, joined to the first face along the edges thereof, a layer of a spongy or absorbing material being interposed therebetween; an outer permeable cloth having the same size and being preferably made of the same fabric as the second face of the support and means joined to said first face for tying said support to said plate of the cannula.

2. The hygienic bib of claim 1, comprising a multilayer pad preferably of the same permeable fabric as said outer cloth having a hole for accomodating the cannula, intended to be placed between the patient's skin and the waterproof face of the rec tangular support.

3. The hygienic bib according to claim 2, wherein said means for tying said support to said plate comprises two laces for a quick tie to the plate of the cannula inserted in the patient's passage, said plate having a slot at each end thereof.

4. The hygienic bib according to claim 3, wherein said laces comprises a piece of VELCRO (TM) velvet, comprising, as well known, a "carpet" of small loops, and a strip made of the matching portion of VELCRO material comprising the hooks engaging the loops, having one end joined to one of said velvet piece, each of said strip being intended to be folded, fitted into the corresponding slot of said plate and then engaged to the same velvet piece to which the end thereof is joined.

5. The hygienic bib according to claim 1, wherein said quick joining means placed on said two strips for tying said bib around said patient's neck, comprises snap fasteners of the male-female type.

FIG.2

FIG.1

FIG. 5

FIG. 4

FIG. 6

FIG.7

FIG.3